Europäisches Patentamt

European Patent Office

Office européen des brevets

(11) Publication number : **0 649 910 A1**

## (12) EUROPEAN PATENT APPLICATION

(21) Application number : 94307751.1

(22) Date of filing : 21.10.94

(51) Int. Cl.$^6$ : **C12Q 1/68**

(30) Priority : **22.10.93 JP 265144/93**

(43) Date of publication of application :
**26.04.95 Bulletin 95/17**

(84) Designated Contracting States :
**CH DE FR GB LI**

(71) Applicant : **SUMITOMO ELECTRIC
INDUSTRIES, LIMITED
5-33, Kitahama 4-chome
Chuo-ku
Osaka 541 (JP)**

(72) Inventor : **Osoegawa, Misako, c/o Osaka
Works of
Sumitomo Electric Ind., Ltd.,
1-3, Shimaya 1-chome
Konohana-ku, Osaka (JP)**

Inventor : **Miyabe, Yuki, c/o Osaka Works of
Sumitomo Electric Ind., Ltd.,
1-3, Shimaya 1-chome
Konohana-ku, Osaka (JP)**
Inventor : **Nakata, Motomi, c/o Osaka Works of
Sumitomo Electric Ind., Ltd.,
1-3, Shimaya 1-chome
Konohana-ku, Osaka (JP)**
Inventor : **Ra, Chisei, c/o Juntendo Univ.
School of Medicine
2-1-1 Hongo,
Bunkyo-ku
Tokyo (JP)**
Inventor : **Suzuki, Katsuhiro, c/o Juntendo Univ
School
of Medicine,
2-1-1 Hongo,
Bunkyo-ku
Tokyo (JP)**

(74) Representative : **Perry, Robert Edward
GILL JENNINGS & EVERY
Broadgate House
7 Eldon Street
London EC2M 7LH (GB)**

(54) DNA probe for detecting allergic disease, and detection process of allergic disease gene making use of the same.

(57) A DNA probe, 10-50 bases long, includes a base sequence selected from the following formulae (1) to (5), and base sequences complementary thereto :
(1) 5' TCC TGC TCT 3'
(2) 5' CTC TCA CCA 3'
(3) 5' ATA TAG GCC 3'
(4) 5' TAC AAT TTC 3' ; and
(5) 5' TGT GAG TGA 3'.
The probe can be used to detect an allergic disease gene, by affinity chromatography.

EP 0 649 910 A1

FIELD OF THE INVENTION

The present invention relates to DNA probes for detecting allergic diseases, and a process for detecting an allergic disease gene making use of such a probe, and more particularly to DNA probes for detecting allergic diseases, by which mutation in a $\beta$ chain gene of an IgE receptor, said mutation being a diathesis of an allergic disease, can be detected, and a process for detecting an allergic disease gene making use of such a DNA probe for detecting an allergic disease.

The DNA probes for detecting allergic diseases according to the present invention can be used in fields of genetic engineering and industries related thereto and are useful particularly in the gene diagnosis of an allergic disease.

BACKGROUND OF THE INVENTION

When an immune response essentially having as its object biophylaxis results in injury to its own vital tissue, such an immune reaction is called an allergic reaction. In general, allergic reactions are classified into 4 types of I to IV types.

Of these, the allergic reaction type I is a typical immediate type allergy or immediate hypersensitivity. An antibody belonging to an IgE class strongly couples to the surface of a mast cell or a basophil at its Fc fragment. When a homologous antigen couples to such an antibody, chemical mediators such as histamine and leukotriene are released by its stimulation. Reactions such as vascular permeability acceleration, angiotelectasis, smooth muscle constriction, muciparous acceleration and leukocyte migration are brought about by the action of these chemical mediators. Such reactions are called immediate allergy because it only takes several minutes to several tens minutes from the entry of the antigen to the expression of symptom.

More specifically, the mast cell has many granules to be stained with a basophilic pigment in its cytoplasm. These granules contain chemical mediators such as histamine therein. An IgE receptor having high affinity for IgE is present on the surface of the mast cell. Immunological specificity, which is a function of the mast cell, is determined by the IgE coupled to the IgE receptor. When IgE molecules coupled to the surface of the mast cell through the IgE receptor are crosslinked with a polyvalent antigen, degranulation takes place, whereby the symptom of an immediate type allergic reaction is caused.

The basophil has basophilic granules containing histamine and heparin in its cytoplasm. As with the mast cell, an IgE receptor having high affinity for IgE is present on the surface of the cell, and chemical mediators such as histamine are released from the basophil by an antigen-antibody reaction.

As specific examples of type I allergic diseases, there have been known anaphylactic shock, bronchial asthma, allergic rhinitis, urticaria, digestive tract allergy, infantile eczema and the like. Many of the group of these diseases have already been described in medical books from the days of Hippocrates, and it has also been considered that there are genetic diatheses therein.

It has recently been indicated that the genetic diathesis of such an allergic disease may possibly be a gene of an IgE receptor $\beta$ chain (Lancet, Vol. 341, Feb. 6, 1993).

However, although it has heretofore been suggested that the allergic diseases correlate to genetic heterogeneity, the heterogeneity of a gene forming the cause has not been found, and DNA probes permitting the gene diagnosis of the allergic diseases have had no existence. It has therefore been difficult to diagnose the allergic diseases at the gene level.

OBJECTS AND SUMMARY OF THE INVENTION

It is an object of the present invention to provide DNA probes for detecting allergic diseases, and a process for detecting an allergic disease gene making use of such a probe.

The present inventors have investigated nucleic acid sequences of genes (expression genes) of IgE receptor $\beta$ chains as to persons of health and cases of allergy to detect the mutations of genes involved in allergic diseases. As a result, it has been found that point mutation is occurring at 5 sites in total in the genes of the IgE receptor $\beta$ chains of the allergic patients compared with the persons of health.

Therefore, respective DNA probes capable of detecting the point mutations of the nucleic acid sequences of the IgE receptor $\beta$ chains have been prepared to detect whether the genes of IgE receptor $\beta$ chains undergo a point mutation or not, using these DNA probes. As a result, it has been revealed that the genetic diatheses of the allergic patients can be directly detected by the DNA probes. The present invention has been led to completion on the basis of these findings.

According to the present invention, there is thus provided a DNA probe for detecting an allergic disease, comprising a base sequence of 10-50 bases in overall length containing one base sequence selected from the group consisting of base sequences represented by the following formulae (1) to (5) and base sequences complementary to these base sequences:

$$(1) \quad 5' \ TCC \ TGC \ TCT \ 3';$$

$$(2) \quad 5' \ CTC \ TCA \ CCA \ 3';$$

$$(3) \quad 5' \ ATA \ TAG \ GCC \ 3';$$

$$(4) \quad 5' \ TAC \ AAT \ TTC \ 3';$$

and

$$(5) \quad 5' \ TGT \ GAG \ TGA \ 3'.$$

According to the present invention, there is also provided a process for detecting an allergic disease gene, which comprises the steps of:

(a) denaturing a DNA fragment specimen containing an IgE receptor gene into a single-stranded DNA fragment,

(b) annealing the single-stranded DNA fragment with the above-described DNA probe specifically coupling to a particular region of the IgE receptor gene to form a conjugate of an objective DNA fragment and the DNA probe,

(c) bringing the conjugate of the objective DNA fragment and the DNA probe into contact with an insoluble support, to which a ligand specifically coupling to the objective DNA fragment has been immobilized, in a column containing the insoluble support as a stationary phase to bond the conjugate to the ligand,

(d) gradually raising the temperature of the column, thereby weakening the bonding strength of the DNA probe and the objective DNA fragment to elute the DNA probe from the column, and

(e) analyzing the elution pattern thus obtained.

## BRIEF DESCRIPTION OF THE DRAWINGS

FIG. 1 illustrates DNA sequences (No. 1 to 400) of respective IgE receptor β chain genes as to a person of health and 3 cases of allergic diseases.

FIG. 2 illustrates DNA sequences (No. 401 to 735) respectively subsequent to the DNA sequences of FIG. 1.

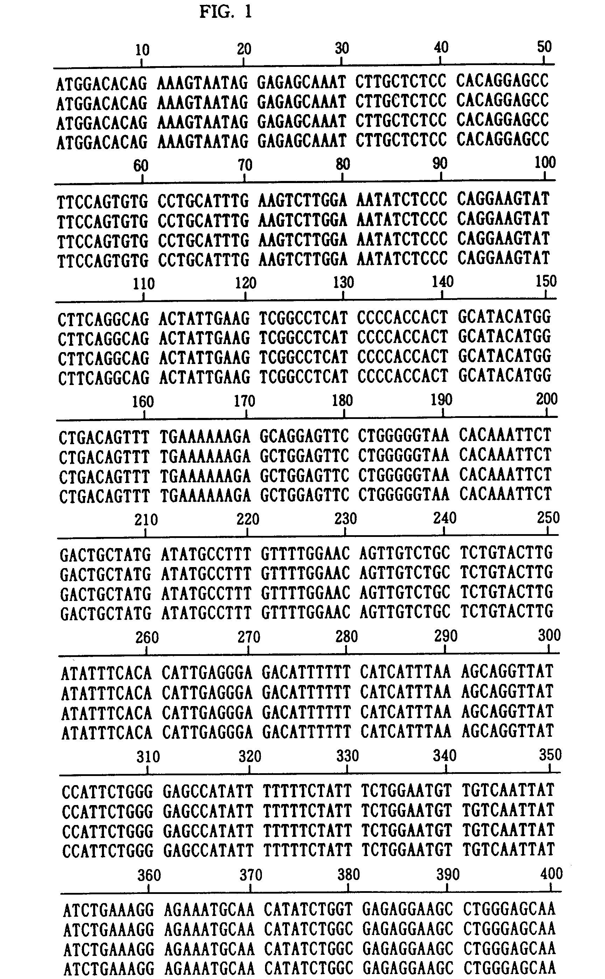

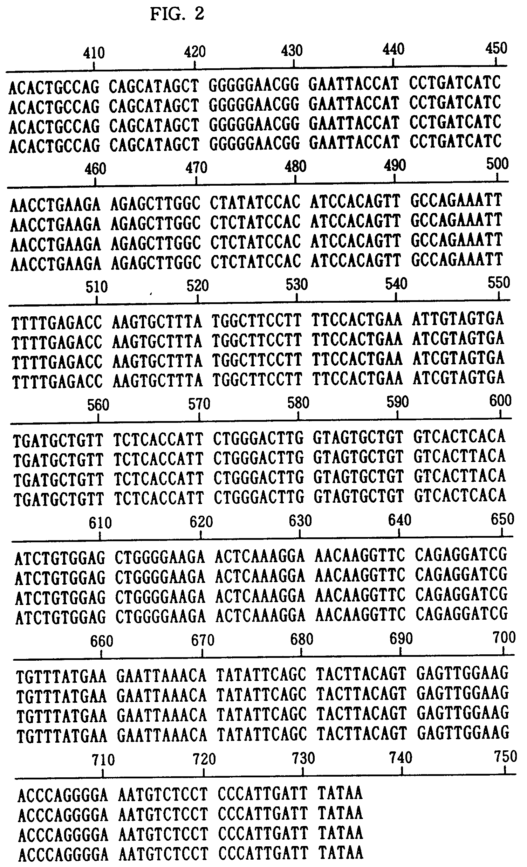

The DNA sequences of FIGs. 1 and 2 are each numbered consecutively from one and upward, beginning with the 5' terminal and ending with the 3' terminal.

## DETAILED DESCRIPTION OF THE INVENTION

The present invention will hereinafter be described in detail.

DNA probes for detecting allergic diseases, which permit the gene diagnosis of the allergic diseases, have heretofore had no existence.

The present inventors have investigated nucleic acid sequences of genes (expression genes) of IgE receptor β chains as to persons of health and cases of allergy to detect the mutation of genes involved in allergic diseases.

First of all, blood was collected from the persons of health and allergic patients to extract respective RNAs from the collected blood cells samples. After each of the thus-obtained RNAs was converted to a cDNA, a gene of an IgE receptor β chain was amplified by PCR (polymerase chain reaction) to sequence this product.

As a result, it was discovered that in the allergic patients, point mutation is occurring in the gene of the IgE receptor β chain at at least one of the following sites:

(1) No. 173 A → T;

(2) No. 380 T → C;

(3) No. 473 A → C;

(4) No. 543 T → C; and

(5) No. 597 C → T.

The DNA sequences of IgE receptor β chain genes of a person of health and 3 allergic patients are illustrated in FIGs. 1 and 2. The point mutations of (1) to (5) and (1) to (4) were found in the genes of the allergic patients 1 and 2 and the gene of the allergic patient 3, respectively.

In the point mutations (1) to (3) among the mutations at this nucleic acid level, the following respective mutations at the amino acid level were also found to be caused:

(1)     Gln (Q) → Arg (R);

(2)     Val (V) → Ala (A);

and

(3)     Tyr (Y) → Ser (S).

With respect to an α chain and a γ chain which are other subunits constituting a high-affinity IgE receptor, whether gene mutation was occurring between the persons of health and the allergic patients or not was also investigated in the same manner as described above. As a result, it was found that no mutation was recognized in the α chain and γ chain of the IgE receptor gene.

On the basis of the above findings, DNA probes capable of detecting the respective point mutations of a gene (nucleic acid sequence) of an IgE receptor β chain were prepared. It was possible to directly detect the genetic diathesis of an allergic disease by detecting the occurrence of a point mutation in the IgE receptor β chain gene using such a DNA probe.

According to the present invention, there are provided DNA probes capable of directly detecting gene mutations correlating to allergies. The DNA probes are intended to separately detect the mutations at 5 sites in the DNA sequence of the IgE receptor β chain gene and separately contain the following base sequences.

(1) The DNA probe for detecting an allergic disease, which detects the mutation (A → T mutation) at a site of No. 173 in the DNA sequence of the IgE receptor β chain gene, is composed of a base sequence of 10-50 bases in length containing the base sequence of TCC TGC TCT or a base sequence complementary to this sequence.

(2) The DNA probe for detecting an allergic disease, which detects the mutation (T → C mutation) at a site of No. 380 in the DNA sequence of the IgE receptor β chain gene, is composed of a base sequence of 10-50 bases in length containing the base sequence of CTC TCA CCA or a base sequence complementary to this sequence.

(3) The DNA probe for detecting an allergic disease, which detects the mutation (A → C mutation) at a site of No. 473 in the DNA sequence of the IgE receptor β chain gene, is composed of a base sequence of 10-50 bases in length containing the base sequence of ATA TAG GCC or a base sequence complementary to this sequence.

(4) The DNA probe for detecting an allergic disease, which detects the mutation (T → C mutation) at a site of No. 543 in the DNA sequence of the IgE receptor β chain gene, is composed of a base sequence of 10-50 bases in length containing the base sequence of TAC AAT TTC or a base sequence complementary to this sequence.

(5) The DNA probe for detecting an allergic disease, which detects the mutation (C → T mutation) at a site of No. 597 in the DNA sequence of the IgE receptor β chain gene, is composed of a base sequence of 10-50 bases in length containing the base sequence of TGT GAG TGA or a base sequence complementary to this sequence.

The DNA probes according to the present invention are synthetic DNAs identical with or complementary to a part of the base sequence of the IgE receptor β chain and composed of 10-50 bases, preferably 15-30 bases in length. The DNA probes are designed so as to contain a base sequence corresponding to a mutating portion in the base sequence of the IgE receptor β chain. In each of the DNA probes, its length is controlled to 10-50 bases, thereby ensuring that whether its corresponding mutation is occurring or not can be detected. It is desirable from the viewpoint of detection accuracy that the design is made so as to locate the base sequence corresponding to the mutating portion in the center of the base sequence of the DNA probe.

For example, the DNA probe for detecting an allergic disease, which detects the mutation (A → T mutation) at a site of No. 173 in the DNA sequence of the IgE receptor β chain gene contains the base sequence of TCC TGC TCT. This sequence is a sequence complementary to the sequence of AGA GC<u>A</u> GGA (<u>A</u> indicating the base of a site undergoing a point mutation) situated at No. 168 to 176 of the DNA sequence illustrated in FIG 1. This DNA probe is constituted in such a manner that the base sequence TCC TGC TCT is located in the center of 10 to 50 bases. For a single-stranded DNA having a sequence complementary to the single-stranded DNA illustrated in FIGs. 1 and 2, a DNA probe having a base sequence complementary to the above-described base sequence is useful.

In general, synthetic oligonucleotides (synthetic DNAs) are used in the DNA probes according to the present invention. Specific examples of preferred DNA probes are set forth in Example which will be described subsequently.

The DNA probes may be used as they are. However, their detection sensitivity can be enhanced by using, for example, a fluorochrome such as fluorescein isothiocyanate (FITC), rhodamine B isothiocyanate (RITC), phycoerythrin (PE) or europium; an enzyme such as peroxydase, alkaline phosphatase, acid phosphatase, $\beta$-galactosidase or cytochrome c; or a hapten such as biotin as a labeling substance to bond it to their 5' terminals.

As a method of bonding the labeling substance to the DNA probe (synthetic DNA), may be mentioned a method in which the functional group (isocyanate group, carboxyl group, amino group or the like) in the labeling substance is reacted with the synthetic DNA, in which a functional group has been introduced, as it is or after it is activated. As a method of detecting the DNA probe labeled with the labeling substance, may be mentioned methods using ultraviolet absorption, fluorescence, chemiluminescence or the like.

In order to detect a gene of an allergic disease with the DNA probe for detecting the allergic disease according to the present invention, various processes may be considered. A specific example of a preferred detection process is a process including the following steps (a) to (e).

(a) A DNA fragment specimen (a DNA sample), which contains an IgE receptor gene, is denatured into a single-stranded DNA fragment.

(b) The thus-prepared single-stranded DNA fragment specimen is annealed with the DNA probe specifically coupling to a particular region of the IgE receptor gene to form a conjugate of an objective DNA fragment (a DNA fragment containing a base sequence of a $\beta$ chain) in the DNA fragment specimen and the DNA probe.

(c) The conjugate of the objective DNA fragment and the DNA probe is brought into contact with an insoluble support, to which a ligand specifically coupling to the objective DNA fragment has been immobilized, to bond the conjugate to the support. This process is generally performed in a column containing, as a stationary phase, the insoluble support with the ligand immobilized thereto.

(d) After DNA fragments not coupled to the insoluble support are washed out, the temperature of the column is gradually raised while supplying a solution serving as a mobile phase into the column, thereby weakening the bonding strength of the objective DNA fragment and the DNA probe to dissociate the DNA probe from the objective DNA and elute it from the column.

(e) The elution pattern thus obtained is analyzed. The bonding strength of the objective DNA fragment and the DNA probe varies with whether a point mutation is occurring in the objective DNA fragment or not. When the point mutation occurs, the bonding strength becomes weak, and so the DNA probe is dissociated and eluted at a lower temperature. Therefore, whether the DNA fragment detected is a gene of an allergic disease or not can be recognized by determining an elution pattern, more specifically, a dissociation temperature.

As the DNA sample (the DNA fragment specimen) used in the detection, a cDNA converted from an RNA extracted from blood or the like, or a DNA is used as it is or after an objective portion is amplified by the PCR. It is preferable to use, as the DNA fragment specimen, a $\beta$ chain gene of a human high-affinity IgE receptor. As the DNA fragment specimens, there may be used cDNAs prepared by extracting mRNAs from human peripheral blood cells of persons of health and allergic patients, converting them to cDNAs and then subjecting the cDNAs to the PCR process using a probe capable of amplifying an IgE receptor $\beta$ chain.

A ligand specifically coupling to a particular single-stranded DNA fragment, which is an objective substance to be detected, is immobilized to the insoluble support. As the ligand to be bonded to the insoluble support, there may be generally used a synthetic DNA identical with or complementary to a part of the base sequence of the IgE receptor $\beta$ chain which is the DNA specimen. In the above-described detection process, a base sequence of a region in which no mutation is occurring, or a base sequence complementary thereto is generally selected from the IgE receptor $\beta$ chain gene to use it as the synthetic DNA serving as the ligand. However, a synthetic DNA composed of a base sequence containing a base of a site at which a mutation other than the particular point mutation to be detected is occurring, or a base sequence complementary thereto may also be used. Namely, a synthetic DNA composed of a base sequence of the IgE receptor $\beta$ chain gene other than the base sequence of the DNA probe to be used, or a base sequence complementary thereto is used. The length of the synthetic DNA serving as the ligand is generally 10-100 bases, preferably 20-50 bases.

As examples of the insoluble support, may be mentioned inorganic materials such as silica gel, porous glass, graphite and apatite; polymeric materials such as polystyrene, nylon, polyethylene, polytetrafluoroethylene, cellulose and nitrocellulose; metals such as aluminum; and ceramics such as alumina. The forms of the insoluble support include beads and a membrane. The insoluble support may be used in the form chemically or physically modified at its surface by, for example, a treatment such as formation of diol, activation with tresyl chloride.

An example of a bonding method of the ligand and the insoluble support includes a method in which various functional groups (for example, carboxyl, amino group, hydroxyl group and the like) introduced in the insoluble support are reacted with the synthetic DNA chemically modified at its terminal by a functional group (amino group, carboxyl group or the like), as they are or after they are subjected to an activation treatment with a condensation agent (water-soluble carbodiimide, carbonyldiimidazole, succinimide or the like) or an activator (tresyl chloride or the like).

In the present invention, the detection of a gene of an allergic disease is conducted using a technique of affinity chromatography in which the insoluble support with the ligand immobilized thereto is used as a stationary phase. In order to promptly conduct the treatment, it is preferable to use high performance liquid chromatography (HPLC). No particular limitation is imposed on a chromato-column so far as it withstands a hydrostatic pressure of generally 100 kg/cm² or higher and is inert to a solution. An inner diameter of the chromato-column is generally 1-20 mm, preferably 1-5 mm. A flow rate of a solution fed to the column is generally 0.01-1 ml/min, preferably 0.05-0.5 ml/min. No particular limitation is imposed on the solution fed to the column so far as it does not attack the insoluble support and the chromato-column. For example, aqueous solutions containing a solute such as phosphoric acid, into which an organic solvent such as methanol, ethanol or acetoaldehyde is mixed, may be exemplified.

In the above-described detection process, the respective base sequences are designed in such a manner that the ligand bonded to the insoluble support specifically couples to a base sequence portion of the objective DNA fragment in which no point mutation is occurring, and the DNA probe specifically couples to a base sequence portion of the objective DNA fragment in which a point mutation is occurring.

With respect to the elution pattern, an elution pattern that the axis of ordinate and the axis of abscissa indicate fluorescence intensity and measuring time, respectively, is obtained, for example, when a DNA probe labeled with a fluorescent substance is used. When the DNA probe is eluted with the elution rate of the column constant, the relation of the elution pattern to a peak temperature is known. If the objective DNA fragment contains a base sequence according to a point mutation, its interaction with the DNA probe becomes weak, and so the DNA probe is eluted at a lower temperature than that in the normal DNA fragment having a base sequence completely complementary to the DNA probe though such a temperature varies with the kind of the mutation. The heating rate of the column is generally 0.1-3°C/min, preferably 0.5-1.0°C/min.

According to the detection process of an allergic disease gene of the present invention, whether a DNA having an objective, particular base sequence exists in a DNA fragment sample or not, more specifically, a mutation is occurring in the base sequence of the objective DNA fragment or not, or the kind of the mutation can be detected.

Other detection processes than the above detection process for detecting the allergic disease gene include, for example, a process in which only an objective DNA fragment (single strand) in a DNA fragment specimen (sample) is specifically bonded to the ligand coupled to the insoluble support, and the objective DNA fragment is then annealed with the DNA probe to specifically bond them to each other. The subsequent process for eluting the DNA probe is as described above.

A further process for detecting the allergic disease gene includes, for example, a process in which the DNA probe is bonded to the insoluble support as a ligand to use it. An example of a bonding method of the DNA probe and the insoluble support includes a method in which various functional groups introduced in the insoluble support are reacted with the DNA probe chemically modified at its terminal by a functional group (amino group, carboxyl group or the like), as they are or after they are subjected to an activation treatment with a condensation agent or an activator. After the DNA fragment specimen (sample) is denatured into a single-stranded DNA fragment, the single-stranded DNA fragment is brought into contact with the insoluble support, to which the DNA probe specifically coupling to the objective DNA fragment has been immobilized, in a column containing the insoluble support as a stationary phase to bond the objective DNA fragment to the DNA probe. Other DNA fragments are then washed out, after which the temperature of the column is gradually raised, thereby weakening the bonding strength of the objective DNA fragment and the DNA probe to elute the DNA fragment from the column. The elution pattern thus obtained is then analyzed. In this process, the objective DNA is eluted, but the DNA probe is not done. Therefore, it is preferable to label the DNA fragment specimen with a labeling substance.

## ADVANTAGES OF THE INVENTION

Since an genetic diathesis of an allergic disease can be detected with ease by using the DNA probe for detecting an allergic disease according to the present invention, it may be used in fields of gene diagnosis, neonatal screening, prenatal diagnosis and the like.

## EMBODIMENTS OF THE INVENTION

The present invention will hereinafter be described in more detail by reference to the following example.

Example 1:

1. Design of DNA probe:

Among the DNA probes according to the present invention, which respectively detect the mutated sites of an IgE receptor β chain gene, those having a length of 24 bases or 25 bases are described by way of example.
(1) An example of a DNA probe for detecting an allergic disease, which detects the mutation at a site of No. 173 (hereinafter referred to as Probe 1):

```
5' CCC AGG AAC TCC TGC TCT TTT TTC A 3';
```

(2) An example of a DNA probe for detecting an allergic disease, which detects the mutation at a site of No. 380 (hereinafter referred to as Probe 2):

```
5' GGC TTC CTC TCA CCA GAT ATG TTG 3';
```

(3) An example of a DNA probe for detecting an allergic disease, which detects the mutation at a site of No. 473 (hereinafter referred to as Probe 3):

```
5' TGG ATG TGG ATA TAG GCC AAG CTC 3';
```

(4) An example of a DNA probe for detecting an allergic disease, which detects the mutation at a site of No. 543 (hereinafter referred to as Probe 4):

```
5' CAT CAT CTC TAC AAT TTC AGT GGA 3';
```

and
(5) An example of a DNA probe for detecting an allergic disease, which detects the mutation at a site of No. 597 (hereinafter referred to as Probe 5):

```
5' TCC ACA GAT TGT GAG TGA CAC AGC A 3'.
```

In the above base sequences, underlined parts indicate bases corresponding to respective mutated sites.

2. Detection of mutation of IgE receptor β chain gene:

2-1. Preparation of detecting probe:

DNAs having the following respective base sequences were synthesized by means of a DNA synthesizer (manufactured by The Perkin-Elmer Corporation), and were labeled in accordance with a method known per se in the art by using the functional group X (X: Aminolink 2, product of ABI Company) situated at the 5' terminal of each DNA to add FITC (fluorescein isothiocyanate) to the terminal.
(1) Probe 1:

```
5' X CCC AGG AAC TCC TGC TCT TTT TTC A 3';
```

(2) Probe 2:

```
5' X GGC TTC CTC TCA CCA GAT ATG TTG 3';
```

(3) Probe 3:

```
5' X TGG ATG TGG ATA TAG GCC AAG CTC 3';
```

(4) Probe 4:

```
5' X CAT CAT CTC TAC AAT TTC AGT GGA 3';
```

and
(5) Probe 5:

$$5' \ X \ TCC \ ACA \ GAT \ TGT \ \underline{G}AG \ TGA \ CAC \ AGC \ A \ 3'.$$

## 2-2. Preparation of DNA-immobilized column:

Fifty bases at the sites of which no mutation occurred were selected from an IgE receptor β chain gene to synthesize a DNA having the following base sequence by the DNA synthesizer.

$$5' \ XCT \ TCA \ GGC \ AGA \ CTA \ TTG \ AAG \ TCG \ GCC \ TCA \ TCC \ CCA$$

$$CCA \ CTG \ CAT \ ACA \ TGG \ 3' \ (X: \ Aminolink \ 2).$$

After 6 mg of this DNA was annealed with an equivalent weight of a complementary strand DNA (protective DNA) in a 300 μl of 1M NaCl, 300 μl of 0.4M NaHCO₃ (pH 7.5) was added thereto, followed by reaction of the conjugate with 300 mg of tresyl chloride-activated silica gel (Nucleosil 1000-OH, particle size: 7 μl, pore size: 1000 Å, product of Nagel Company) for 24 hours. After completion of the reaction, an excess amount of the DNA was removed, and the reaction product was then washed with 2.4M of TEACl at 65°C, thereby dissociating the double-stranded DNA into a single-stranded DNA to prepare a DNA-immobilized support.

This DNA-immobilized support was suspended in a packing solution (0.5M NaCl, 10mM phosphoric acid, 1mM EDTA, pH 7.0) to place it in a packer. The packing solution was then fed to a column (2.1 mm across x 10 cm long) to fill the support in the column.

## 3. Preparation of DNA specimen by PCR:

After an RNA was extracted from 10 ml of human peripheral blood in accordance with a method known per se in the art, the RNA was converted to a cDNA sample by a treatment with an enzyme, thereby subjecting the cDNA sample to the PCR under the following conditions.

| 10 x Buffer | 10 μl |
|---|---|
| 5' Primer (20μM) | 5 μl |
| 3' Primer (20μM) | 5 μl |
| dNTPs (each 2.5mM) | 8 μl |
| ADDW (Autoclave double distilled water) | 46 μl |
| cDNA | 25 μl |
| Thermostable DNA polymerase | 1 μl |
| | 100 μl |

| Temperature Cycle 1: | 94°C | 5 min |
|---|---|---|
| Temperature Cycle 2: | 94°C | 1 min |
| | 55°C | 1 min |
| | 72°C | 2 min |
| (Repeating Temperature Cycle 2 30 times) | | |
| Temperature Cycle 3: | 55°C | 2 min |
| | 72°C | 10 min |

Details of the above materials used in the PCR are as follows:

<PCR 5' Primer>

5' CTG CTC GAG ATG GAC ACA GAA AGT AAT AGG 3';

<PCR 3' Primer>

5' CAT GGC GGC CGC TTA TAA ATA ATC AAT GGG AGG AGA

CAT TTC CCC 3';

<Thermostable DNA polymerase>

AmpliTaq DNAPolymerase, trade name, product of Roche Molecular Systems, Inc.

4. Analysis of DNA specimen by column:

The above-prepared DNA-immobilized column was connected to a high performance liquid chromatograph (manufactured by Waters Company) (solution: 40% ethanol, 10mM phosphoric acid, 1mM EDTA).

After 10 µl of the product (the DNA fragment specimen) by the PCR was thermally denatured to dissociate it into a single-stranded DNA fragment, the DNA probe for detection was mixed with the single-stranded DNA fragment to anneal them. The resulting conjugate was injected into the column kept at 30°C.

After the solution was caused to flow through the column for 10 minutes to wash the interior of the column, the temperature of the column was raised from 30°C to 60°C at a heating rate of 0.5°C/min to detect the detector probe eluted by means of a fluorometer. The dissociation temperature was determined from the elution time of the detector DNA probe. The above-described experiment was performed on the respective detector DNA probes. The results are shown in Table 1.

Table 1

|  | Dissociation temperature on normal gene (°C) | Dissociation temperature on mutational gene (°C) |
|---|---|---|
| Probe 1 | 42.0 | 40.0 |
| Probe 2 | 41.0 | 35.0 |
| Probe 3 | 40.5 | 36.0 |
| Probe 4 | 38.0 | 36.5 |
| Probe 5 | 43.0 | 41.0 |

As apparent from the results shown in Table 1, genes of allergic diseases can be detected from the dissociation temperatures of the respective detector DNA probes.

## Claims

1. A DNA probe, 10-50 bases long, including a base sequence selected from the following formulae (1) to (5), and base sequences complementary thereto:

$$(1) \quad 5' \ \text{TCC TGC TCT} \ 3'$$

$$(2) \quad 5' \ \text{CTC TCA CCA} \ 3'$$

$$(3) \quad 5' \ \text{ATA TAG GCC} \ 3'$$

$$(4) \quad 5' \ \text{TAC AAT TTC} \ 3';$$

and

$$(5) \quad 5' \ \text{TGT GAG TGA} \ 3'.$$

2. A DNA probe according to claim 1, which has a base sequence identical or complementary to a part of the base sequence (a single-stranded DNA) of a β chain gene of a human high-affinity IgE receptor.

3. A DNA probe according to claim 1 or claim 2, additionally comprising a terminal label.

4. A DNA probe according to any preceding claim, which is immobilised by a terminal bond to an insoluble support.

5. A DNA probe according to any preceding claim, including the base sequence of any of SEQ ID NOS. 1 to 5.

6. A process for detecting an allergic disease gene, which comprises the steps of:
   (a) denaturing a DNA fragment specimen containing an IgE receptor gene, give a single-stranded DNA fragment;
   (b) annealing the single-stranded DNA fragment with a DNA probe according to any preceding claim, and specifically coupling to a particular region of the IgE receptor gene, to form a conjugate of an objective DNA fragment and the DNA probe;
   (c) bringing the conjugate into contact with an insoluble support, to which a ligand specifically coupling to the objective DNA fragment has been immobilised, in a column containing the insoluble support as a stationary phase, to bond the conjugate to the ligand;
   (d) gradually raising the temperature of the column, thereby weakening the conjugate, to elute the DNA probe from the column, and
   (e) analysing the elution pattern thus obtained.

7. A process according to claim 6, wherein the DNA fragment specimen (i) contains a β chain gene of a human high-affinity IgE receptor, or (ii) is obtainable by extracting a mRNA from human peripheral blood cells of a healthy person or an allergic patient, converting it to a cDNA and then subjecting the cDNA to a polymerase chain reaction (PCR) using a probe capable of amplifying an IgE receptor β chain.

8. A process according to claim 6 or claim 7, wherein the ligand is a synthetic DNA, 10-100 bases long, composed of a base sequence of the IgE receptor β chain gene other than the base sequence of the DNA probe, e.g. of SEQ ID NO. 6, or a base sequence complementary thereto.

9. A process according to any of claims 6 to 8, wherein the insoluble support is tresyl chloride-activated silica gel.

10. A process according to any of claims 6 to 9, wherein the analysing comprises recognising an allergic disease gene if the dissociation temperature of the DNA probe read from the elution pattern is different from the corresponding case where a DNA fragment specimen containing a normal IgE receptor β chain gene is used.

FIG. 1

```
          10        20        30        40        50
          |         |         |         |         |
ATGGACACAG AAAGTAATAG GAGAGCAAAT CTTGCTCTCC CACAGGAGCC
ATGGACACAG AAAGTAATAG GAGAGCAAAT CTTGCTCTCC CACAGGAGCC
ATGGACACAG AAAGTAATAG GAGAGCAAAT CTTGCTCTCC CACAGGAGCC
ATGGACACAG AAAGTAATAG GAGAGCAAAT CTTGCTCTCC CACAGGAGCC

          60        70        80        90        100
          |         |         |         |         |
TTCCAGTGTG CCTGCATTTG AAGTCTTGGA AATATCTCCC CAGGAAGTAT
TTCCAGTGTG CCTGCATTTG AAGTCTTGGA AATATCTCCC CAGGAAGTAT
TTCCAGTGTG CCTGCATTTG AAGTCTTGGA AATATCTCCC CAGGAAGTAT
TTCCAGTGTG CCTGCATTTG AAGTCTTGGA AATATCTCCC CAGGAAGTAT

          110       120       130       140       150
          |         |         |         |         |
CTTCAGGCAG ACTATTGAAG TCGGCCTCAT CCCCACCACT GCATACATGG
CTTCAGGCAG ACTATTGAAG TCGGCCTCAT CCCCACCACT GCATACATGG
CTTCAGGCAG ACTATTGAAG TCGGCCTCAT CCCCACCACT GCATACATGG
CTTCAGGCAG ACTATTGAAG TCGGCCTCAT CCCCACCACT GCATACATGG

          160       170       180       190       200
          |         |         |         |         |
CTGACAGTTT TGAAAAAAGA GCAGGAGTTC CTGGGGGGTAA CACAAATTCT
CTGACAGTTT TGAAAAAAGA GCTGGAGTTC CTGGGGGGTAA CACAAATTCT
CTGACAGTTT TGAAAAAAGA GCTGGAGTTC CTGGGGGGTAA CACAAATTCT
CTGACAGTTT TGAAAAAAGA GCTGGAGTTC CTGGGGGGTAA CACAAATTCT

          210       220       230       240       250
          |         |         |         |         |
GACTGCTATG ATATGCCTTT GTTTTGGAAC AGTTGTCTGC TCTGTACTTG
GACTGCTATG ATATGCCTTT GTTTTGGAAC AGTTGTCTGC TCTGTACTTG
GACTGCTATG ATATGCCTTT GTTTTGGAAC AGTTGTCTGC TCTGTACTTG
GACTGCTATG ATATGCCTTT GTTTTGGAAC AGTTGTCTGC TCTGTACTTG

          260       270       280       290       300
          |         |         |         |         |
ATATTTCACA CATTGAGGGA GACATTTTTT CATCATTTAA AGCAGGTTAT
ATATTTCACA CATTGAGGGA GACATTTTTT CATCATTTAA AGCAGGTTAT
ATATTTCACA CATTGAGGGA GACATTTTTT CATCATTTAA AGCAGGTTAT
ATATTTCACA CATTGAGGGA GACATTTTTT CATCATTTAA AGCAGGTTAT

          310       320       330       340       350
          |         |         |         |         |
CCATTCTGGG GAGCCATATT TTTTTCTATT TCTGGAATGT TGTCAATTAT
CCATTCTGGG GAGCCATATT TTTTTCTATT TCTGGAATGT TGTCAATTAT
CCATTCTGGG GAGCCATATT TTTTTCTATT TCTGGAATGT TGTCAATTAT
CCATTCTGGG GAGCCATATT TTTTTCTATT TCTGGAATGT TGTCAATTAT

          360       370       380       390       400
          |         |         |         |         |
ATCTGAAAGG AGAAATGCAA CATATCTGGT GAGAGGAAGC CTGGGAGCAA
ATCTGAAAGG AGAAATGCAA CATATCTGGC GAGAGGAAGC CTGGGAGCAA
ATCTGAAAGG AGAAATGCAA CATATCTGGC GAGAGGAAGC CTGGGAGCAA
ATCTGAAAGG AGAAATGCAA CATATCTGGC GAGAGGAAGC CTGGGAGCAA
```

## FIG. 2

```
           410        420        430        440        450
ACACTGCCAG CAGCATAGCT GGGGGAACGG GAATTACCAT CCTGATCATC
ACACTGCCAG CAGCATAGCT GGGGGAACGG GAATTACCAT CCTGATCATC
ACACTGCCAG CAGCATAGCT GGGGGAACGG GAATTACCAT CCTGATCATC
ACACTGCCAG CAGCATAGCT GGGGGAACGG GAATTACCAT CCTGATCATC
           460        470        480        490        500
AACCTGAAGA AGAGCTTGGC CTATATCCAC ATCCACAGTT GCCAGAAATT
AACCTGAAGA AGAGCTTGGC CTCTATCCAC ATCCACAGTT GCCAGAAATT
AACCTGAAGA AGAGCTTGGC CTCTATCCAC ATCCACAGTT GCCAGAAATT
AACCTGAAGA AGAGCTTGGC CTCTATCCAC ATCCACAGTT GCCAGAAATT
           510        520        530        540        550
TTTTGAGACC AAGTGCTTTA TGGCTTCCTT TTCCACTGAA ATTGTAGTGA
TTTTGAGACC AAGTGCTTTA TGGCTTCCTT TTCCACTGAA ATCGTAGTGA
TTTTGAGACC AAGTGCTTTA TGGCTTCCTT TTCCACTGAA ATCGTAGTGA
TTTTGAGACC AAGTGCTTTA TGGCTTCCTT TTCCACTGAA ATCGTAGTGA
           560        570        580        590        600
TGATGCTGTT TCTCACCATT CTGGGACTTG GTAGTGCTGT GTCACTCACA
TGATGCTGTT TCTCACCATT CTGGGACTTG GTAGTGCTGT GTCACTTACA
TGATGCTGTT TCTCACCATT CTGGGACTTG GTAGTGCTGT GTCACTTACA
TGATGCTGTT TCTCACCATT CTGGGACTTG GTAGTGCTGT GTCACTCACA
           610        620        630        640        650
ATCTGTGGAG CTGGGGAAGA ACTCAAAGGA AACAAGGTTC CAGAGGATCG
ATCTGTGGAG CTGGGGAAGA ACTCAAAGGA AACAAGGTTC CAGAGGATCG
ATCTGTGGAG CTGGGGAAGA ACTCAAAGGA AACAAGGTTC CAGAGGATCG
ATCTGTGGAG CTGGGGAAGA ACTCAAAGGA AACAAGGTTC CAGAGGATCG
           660        670        680        690        700
TGTTTATGAA GAATTAAACA TATATTCAGC TACTTACAGT GAGTTGGAAG
TGTTTATGAA GAATTAAACA TATATTCAGC TACTTACAGT GAGTTGGAAG
TGTTTATGAA GAATTAAACA TATATTCAGC TACTTACAGT GAGTTGGAAG
TGTTTATGAA GAATTAAACA TATATTCAGC TACTTACAGT GAGTTGGAAG
           710        720        730        740        750
ACCCAGGGGA AATGTCTCCT CCCATTGATT TATAA
ACCCAGGGGA AATGTCTCCT CCCATTGATT TATAA
ACCCAGGGGA AATGTCTCCT CCCATTGATT TATAA
ACCCAGGGGA AATGTCTCCT CCCATTGATT TATAA
```

EP 0 649 910 A1

| | European Patent Office | **EUROPEAN SEARCH REPORT** | Application Number EP 94 30 7751 |

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | FEBS LETT., vol.302, no.2, May 1992, ELSEVIER PUBLISHERS, AMSTERDAM, NL; pages 161 - 165 K. MAEKAWA ET AL. 'Determination of the sequence coding for the beta-subunit of the human high-affinity IgE receptor' | 1,2,5 | C12Q1/68 |
| Y | * see primer EBH5 (nucleotide 532-551) * * page 162, left column, line 7; figures 1,4 * | 6-8 | |
| X | WO-A-88 09376 (GENENTECH, INC.) 1 December 1988 * see brf.1 * * page 33, line 29 - page 34, line 5; figure 2 * | 1,3 | |
| Y | EP-A-0 265 244 (AMOCO CORPORATION) 27 April 1988 * the whole document * | 6-8 | |
| A | J. BIOL. CHEM., vol.267, no.18, 25 June 1992, AM. SOC. MOL. BIOL., INC.,BALTIMORE, US; pages 12782 - 12787 H. KÜSTER ET AL. 'The gene and cDNA for the human high affinity immunglobulin E receptor beta chain and expression of the complete human receptor' * the whole document * | 1-10 | TECHNICAL FIELDS SEARCHED (Int.Cl.6) C12Q |

-/--

The present search report has been drawn up for all claims

| Place of search | Date of completion of the search | Examiner |
|---|---|---|
| THE HAGUE | 3 February 1995 | Hornig, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

16

European Patent
Office

**EUROPEAN SEARCH REPORT**

Application Number

EP 94 30 7751

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | THE LANCET, vol.340, no.8816, 15 August 1992, THE LANCET LTD., LONDON, GB; pages 381 - 384 W.O.C.M. COOKSON ET AL. 'Maternal inheritance of atopic IgE responsiveness on chromosome 11q' * the whole document * | 1-10 | |
| A | THE LANCET, vol.341, no.8841, 6 February 1993, THE LANCET LTD., LONDON, GB; pages 332 - 334 A.J. SANDFORD ET AL. 'Localisation of atopy and beta subunit of high-affinity IgE receptor (FcepsilonRI) on chromosome 11q' | 1-10 | |
| A | EP-A-0 269 451 (SCLAVO, INC.) 1 June 1988 * the whole document * | 9 | |

TECHNICAL FIELDS
SEARCHED    (Int.Cl.6)

The present search report has been drawn up for all claims

| Place of search | Date of completion of the search | Examiner |
|---|---|---|
| THE HAGUE | 3 February 1995 | Hornig, H |

EPO FORM 1503 03.82 (P04C01)

SEQUENCE LISTING

(1) GENERAL INFORMATION:

    (i) APPLICANT:
        (A) NAME: Sumitomo Electric Industries, Ltd.
        (B) STREET: 5-33, Kitahama 4-chome
        (C) CITY: Chuo-ku
        (D) STATE: Osaka
        (E) COUNTRY: Japan
        (F) POSTAL CODE (ZIP): -

    (ii) TITLE OF INVENTION: DNA PROBE FOR DETECTING ALLERGIC DISEASE, AND
        DETECTION PROCESS OF ALLERGIC DISEASE GENE MAKING USE OF
        THE SAME

    (iii) NUMBER OF SEQUENCES: 8

    (iv) COMPUTER READABLE FORM:
        (A) MEDIUM TYPE: Floppy disk
        (B) COMPUTER: IBM PC compatible
        (C) OPERATING SYSTEM: PC-DOS/MS-DOS
        (D) SOFTWARE: PatentIn Release #1.0, Version #1.25 (EPO)


(2) INFORMATION FOR SEQ ID NO: 1:

    (i) SEQUENCE CHARACTERISTICS:
        (A) LENGTH: 25 base pairs
        (B) TYPE: nucleic acid
        (C) STRANDEDNESS: single
        (D) TOPOLOGY: linear

    (ii) MOLECULE TYPE: cDNA

    (iii) HYPOTHETICAL: NO

    (iii) ANTI-SENSE: NO


    (xi) SEQUENCE DESCRIPTION: SEQ ID NO: 1:

CCCAGGAACT CCTGCTCTTT TTTCA                         25


(2) INFORMATION FOR SEQ ID NO: 2:

    (i) SEQUENCE CHARACTERISTICS:
        (A) LENGTH: 24 base pairs
        (B) TYPE: nucleic acid
        (C) STRANDEDNESS: single
        (D) TOPOLOGY: linear

    (ii) MOLECULE TYPE: cDNA

    (iii) HYPOTHETICAL: NO

    (iii) ANTI-SENSE: NO


    (xi) SEQUENCE DESCRIPTION: SEQ ID NO: 2:

GGCTTCCTCT CACCAGATAT GTTG                         24

(2) INFORMATION FOR SEQ ID NO: 3:

    (i) SEQUENCE CHARACTERISTICS:
        (A) LENGTH: 24 base pairs
        (B) TYPE: nucleic acid
        (C) STRANDEDNESS: single
        (D) TOPOLOGY: linear

    (ii) MOLECULE TYPE: cDNA

    (iii) HYPOTHETICAL: NO

    (iii) ANTI-SENSE: NO

    (xi) SEQUENCE DESCRIPTION: SEQ ID NO: 3:

TGGATGTGGA TATAGGCCAA GCTC                24

(2) INFORMATION FOR SEQ ID NO: 4:

    (i) SEQUENCE CHARACTERISTICS:
        (A) LENGTH: 24 base pairs
        (B) TYPE: nucleic acid
        (C) STRANDEDNESS: single
        (D) TOPOLOGY: linear

    (ii) MOLECULE TYPE: cDNA

    (iii) HYPOTHETICAL: NO

    (iii) ANTI-SENSE: NO

    (xi) SEQUENCE DESCRIPTION: SEQ ID NO: 4:

CATCATCTCT ACAATTTCAG TGGA                24

(2) INFORMATION FOR SEQ ID NO: 5:

    (i) SEQUENCE CHARACTERISTICS:
        (A) LENGTH: 25 base pairs
        (B) TYPE: nucleic acid
        (C) STRANDEDNESS: single
        (D) TOPOLOGY: linear

    (ii) MOLECULE TYPE: cDNA

    (iii) HYPOTHETICAL: NO

    (iii) ANTI-SENSE: NO

    (xi) SEQUENCE DESCRIPTION: SEQ ID NO: 5:

TCCACAGATT GTGAGTGACA CAGCA               25

(2) INFORMATION FOR SEQ ID NO: 6:

    (i) SEQUENCE CHARACTERISTICS:
        (A) LENGTH: 50 base pairs
        (B) TYPE: nucleic acid
        (C) STRANDEDNESS: single
        (D) TOPOLOGY: linear

    (ii) MOLECULE TYPE: cDNA

    (iii) HYPOTHETICAL: NO

    (iii) ANTI-SENSE: NO


    (xi) SEQUENCE DESCRIPTION: SEQ ID NO: 6:

CTTCAGGCAG ACTATTGAAG TCGGCCTCAT CCCCACCACT GCATACATGG        50


(2) INFORMATION FOR SEQ ID NO: 7:

    (i) SEQUENCE CHARACTERISTICS:
        (A) LENGTH: 30 base pairs
        (B) TYPE: nucleic acid
        (C) STRANDEDNESS: single
        (D) TOPOLOGY: linear

    (ii) MOLECULE TYPE: cDNA

    (iii) HYPOTHETICAL: NO

    (iii) ANTI-SENSE: NO


    (xi) SEQUENCE DESCRIPTION: SEQ ID NO: 7:

CTGCTCGAGA TGGACACAGA AAGTAATAGG        30


(2) INFORMATION FOR SEQ ID NO: 8:

    (i) SEQUENCE CHARACTERISTICS:
        (A) LENGTH: 45 base pairs
        (B) TYPE: nucleic acid
        (C) STRANDEDNESS: single
        (D) TOPOLOGY: linear

    (ii) MOLECULE TYPE: cDNA

    (iii) HYPOTHETICAL: NO

    (iii) ANTI-SENSE: NO


    (xi) SEQUENCE DESCRIPTION: SEQ ID NO: 8:

CATGGCGGCC GCTTATAAAT AATCAATGGG AGGAGACATT TCCCC        45